# EUROPEAN PATENT SPECIFICATION

(11) **EP 0 563 269 B1**
(45) Date of publication and mention of the grant of the patent: **12.10.1994**
(21) Application number: 92903216.7
(22) Date of filing: 05.12.1991
(51) Int. Cl.: C12P 19/62

(54) **NATAMYCIN RECOVERY**
NATAMYCINGEWINNUNG
PROCEDE DE RECUPERATION DE NATAMYCINE

(30) Priority: 07.12.1990 US 623585
(43) Date of publication of application: 06.10.1993
(73) Proprietor: E.I. DU PONT DE NEMOURS AND COMPANY, Wilmington Delaware 19898 (US)
(72) Inventor: MILLIS, James, R., Kohler, WI 53044 (US); OLSON, Phillip, Terry, Manitowoc, WI 54220 (US); REIMER, Michael, Henry, Sheboygan, WI 53081 (US)
(74) Representative: Woodcraft, David Charles
(86) International application number: US9108931
(87) International publication number: WO9210580

(56) References cited:
- FR-A- 1 188 428
- US-A- 3 378 441
- US-A- 4 006 222

## Description

### BACKGROUND OF THE INVENTION

This invention relates to a process of recovery of natamycin from a fermentation broth containing natamycin by adding methanol and adjusting the pH to 1.0 to 4.5 to dissolve the precipitated natamycin; removing the remaining suspended solids; and raising the pH to 6.0 to 9.0 to precipitate high purity natamycin.

Natamycin is an antibiotic that has been known for decades (Florey, "Analytical Profiles of Drug Substances", Vol. 10, 1981; Merck Index, "Pimaricin", p 834). Despite its recognized valuable antibiotic properties, there has been little research or commercialization of natamycin because of the extremely high cost of its manufacture. This invention relates to an inexpensive, rapid process for recovering natamycin from a fermentation broth.

As is pointed out in Florey, natamycin has a well-defined specific chemical structure, melting point and ultraviolet spectrum. Its specific physico-chemical properties, particularly its solubility in various liquids, require processing techniques specific to this compound. Prior processes for natamycin have not been generally economically acceptable. Processes for recovery of other compounds having different properties give little insight into the recovery of natamycin.

Natamycin has been prepared by fermentation such as disclosed in British Patent 846,933 (American Cyanamid) using Streptomyces gilvosporeus. In this patent, the natamycin is recovered by methanol extraction followed by tedious steps of adsorption and elution.

U.S. Patent 3,378,441 (Penick) discloses recovering natamycin by salting it out of the fermentation broth, extracting with methanol, removing the solids, and then evaporating the liquid to recover the natamycin.

U.S. Patent 3,892,850 (Gist-Brocades) discloses recovery of pimaricin by extraction with acidified butanol followed by distillation and precipitation. It is noted that the pimaricin identification in this patent is inconsistent with those of natamycin as disclosed in Florey.

All of these processes require an expensive ultimate recovery step such as absorption and elution, distillation, or evaporation of liquids.

### SUMMARY OF THE INVENTION

It has been discovered that high purity natamycin can be recovered from fermentation broth containing natamycin by simple extraction with methanol extraction liquid under controlled pH conditions. The present process comprises the steps:
1. adding methanol to a natamycin fermentation product containing precipitated natamycin, the fermentation product plus extraction liquid containing at least 2 g/l natamycin;
2. adjusting the pH to 1.0 to 4.5 to solubilize the natamycin;
3. removing the remaining suspended solids; and
4. raising the pH to 6.0-9.0 to precipitated natamycin.
Following step 4, normally the natamycin is mechanically removed from the liquid such as by filtration or centrifugation, then washed with water and dried yielding natamycin of over 80% purity and usually over 90% purity.

In steps 2 and 4, sufficient time is allowed to approach stable solubility conditions, usually 15-45 minutes being sufficient depending on the volume of the broth, solids concentration, vessel configuration and the like. Agitation may be helpful.

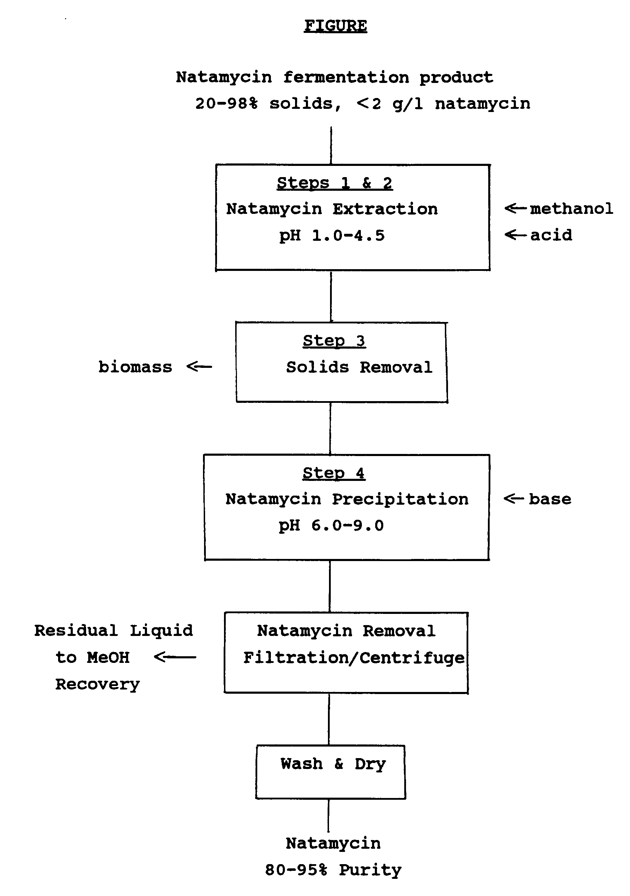

### BRIEF DESCRIPTION OF THE DRAWING

The Figure is a schematic representation of the process of the invention.

### DETAILED DESCRIPTION OF THE INVENTION

The process of the invention requires that the fermentation product plus the extracting liquid contain at least 2 g/l natamycin. If the direct fermentation broth contains too little natamycin water must be removed. Since the fermentation product consists essentially of water and natamycin plus biomass solids, water can be removed by any convenient solid-liquid separation technique, such as filtering and/or centrifuging off water. Concentrations of up to 50% solids is achievable this way. If a higher solids content is desired in the recovery feed stream to minimize the required methanol, heat assisted vacuum evaporation may be used. Typically a temperature in the range 30°C to 80°C is used. Prolonged use of temperatures above 95°C should be avoided to prevent possible product or biomass degradation.

The fermentation product used as the feed stock in the present process comprises solid suspended natamycin and biomass and water. The solids content may be as low as 20% or less, for example, if no water is removed from the fermentation broth. If water must be removed to obtain a fermentation product plus extractant containing at least 2 g/l natamycin, only the minimum water need be removed. Alternatively, depending on equipment availability, particularly for methanol recovery, it may be desirable to remove substantially all of the water before addition of the methanol extractant.

For optimum natamycin recovery, the concentration of natamycin can be as low as 2 g/l natamycin. However, as the water content of the feed stream is decreased, higher concentrations of natamycin are desirable because of higher solubility of it in methanol containing lower amounts of water. Thus, where the solids content of the feed stream is over about 50%, the natamycin content preferably is at least 6 g/l.

The Figure schematically shows the process of this invention as hereinafter described.

In step 1, methanol is used as the extraction medium because natamycin has excellent solubility in this inexpensive solvent in comparison to higher alcohols, and because simple techniques for recovery of methanol are well known. To assure optimum natamycin recovery, methanol is used in the range of 20 g to about 150 g natamycin per liter of methanol in the feed stream. Since natamycin tends to precipitate out of the acidified methanol/water extraction medium at high concentrations, lower concentration in the range of 20 g to 120 g natamycin per liter methanol is preferred. Again because of decreased solubility of natamycin, as the water content of the methanol extraction liquid increases greater quantities of methanol are needed to achieve the same recovery. For example, if the feed contains 70% water, about three times as much methanol is required than when starting with a substantially dry feed stream. If the feed contains 40% water, twice as much methanol is needed.

In step 2, the pH is adjusted to 1.0-4.5 by the addition of an acidifying agent at any stage, such as directly to the methanol or simultaneously with or after the methanol addition directly to the fermentation product. This renders the natamycin highly soluble in the methanol solvent. Hydrochloric acid is an excellent acidifying agent, although any conventional compatible acidic material is acceptable.

Under some circumstances it may be desired to also add an inorganic salt to assist solubilizing although this is normally not required. Typical salts that can be used are NaCl, CaCl₂ and others such as disclosed in Penick U.S. Patent 3,378,441.

In step 3, the remaining biomass suspended solids are removed by any convenient means such as filtration or centrifugation. These solids, which still contain a significant quantity of natamycin can be put to other valuable uses.

In step 4, any compatible basic material can be used to raise the methanol/water/natamycin liquid pH to 6.0-9.0. This pH modification causes precipitation of high purity natamycin, which is removed and dried. Typical useful inexpensive compatible basic materials are sodium and potassium hydroxides. If desired, water may also be added in step 4 to assist in natamycin precipitation. This may be necessary where the water feed stream concentration is low and natamycin concentration is high. The remaining liquid by-product which contains valuable natamycin, fermentation residues, methanol, inorganic salts and water is sent to methanol recovery. Natamycin of about 80% purity can be recovered in the methanol recovery process.

### EXAMPLE 1

This example describes the use of substantially water free fermentation product as the feed stock of the present process.

Fermentation broth is concentrated to 98% solids (plus a small quantity of nutritive residues) by centrifuging followed by drying at 40°C under vacuum. Solids containing about 13 g natamycin are slurried in 240 ml of methanol and the pH is reduced to 3.8 by the addition of concentrated hydrochloric acid. The slurry is mixed for 30 minutes to assist in dissolving natamycin. The suspended solids (biomass and some natamycin) is removed by filtration.

The clarified liquid, consisting essentially of 61 g/l natamycin, methanol and a small quantity of water is raised to pH 7.5 by the addition of potassium hydroxide, and let stand for 30 minutes. A thick white precipitate of natamycin forms. The solids are separated by filtration, washed with water and dried at 40°C under vacuum.

9.3 g of solids are obtained which is 93% purity natamycin (on an anhydrous basis).

### EXAMPLE 2

This example describes the use of fermentation product concentration solely by filtration.

Fermentation broth is concentrated by filtration to 50% solids. This feed stock containing about 13 g natamycin is slurried in 500 ml of methanol.

Thereafter, the procedure of Example 1 is followed except acetic acid and NaOH are used as the acidic and basic pH adjusting additives.

About 9.5 g of natamycin of over 90% purity is obtained.

### EXAMPLE 3

This example describes the use of a low solids content fermentation product feed stock.

Fermentation broth is concentrated by simple mechanical filtration to 23% solids. This fermentation product containing about 2.9 g natamycin is slurried with 40 ml of methanol and the pH reduced to 2.2 by addition of hydrochloric acid. This slurry is agitated for 30 minutes and then the solids are removed by centrifugation followed by filtration.

The pH of the clarified liquid containing 38 g/l natamycin is raised to 7.5 by addition of potassium hydroxide and let stand for 30 minutes. A thick white precipitate forms. The solids are separated out by filtration, washed with water, and dried under vacuum. The dry product is 92% pure natamycin.

3.6 g/l natamycin remains in the liquid, which liquid is sent to methanol recovery where the natamycin is recovered at about 80% purity.

## Claims

1. The process for the recovery of natamycin from a fermentation broth product containing at least 2 g/l natamycin comprising the following steps:
a. adding to said broth product methanol;
b. adjusting the pH to 1.0 to 4.5 to solubilize natamycin;
c. removing the suspended solids; and
d. raising the pH to 6.0-9.0 to precipitate natamycin.

2. The process of Claim 1 wherein the precipitated natamycin is recovered and dried.

3. The process of Claim 1 wherein the fermentation broth product contains 20 to 98% solids.

4. The process of Claim 1 wherein the fermentation product contains at least 50% solids.

5. The process of Claim 1 wherein the methanol is added in an amount of 1 liter methanol per 20 g to 150 g of natamycin in the fermentation broth product.

6. The process of Claim 1 wherein the methanol is added in an amount of 1 liter methanol per 20 g to 120 g of natamycin in the fermentation broth product.

7. The process of Claim 1 wherein water is added in step 4.

8. The process of Claim 1 wherein the fermentation broth product is prepared by removal of water from the direct fermentation broth.

9. The process of Claim 8 wherein said removal of water is done by mechanical means.

## Patentansprüche

1. Verfahren zur Gewinnung von Natamycin aus einem Produkt eines Fermentationsmediums, das wenigstens 2 g/l Natamycin enthält, umfassend die folgenden Stufen:
a. Zugeben von Methanol zu dem genannten Produkt des Nährmediums,
b. Einstellen des pH-Wertes auf 1,0 bis 4,5, um Natamycin in Lösung zu bringen,
c. Entfernen der suspendierten Feststoffe und
d. Erhöhen des pH-Wertes auf 6,0 bis 9,0, um Natamycin auszufällen.

2. Verfahren nach Anspruch 1, bei dem das ausgefällte Natamycin gewonnen und getrocknet wird.

3. Verfahren nach Anspruch 1, bei dem das Produkt des Fermentationsmediums 20 bis 98 % Feststoffe enthält.

4. Verfahren nach Anspruch 1, bei dem das Produkt des Fermentationsmediums wenigstens 50 % Feststoffe enthält.

5. Verfahren nach Anspruch 1, bei dem Methanol in einer Menge von 1 l Methanol auf 20 g bis 150 g Natamycin in dem Produkt des Fermentationsmediums hinzugegeben wird.

6. Verfahren nach Anspruch 1, bei dem das Methanol in einer Menge von 1 l Methanol auf 20 g bis 120g Natamycin in dem Produkt des Fermentationsmediums hinzugegeben wird.

7. Verfahren nach Anspruch 1, bei dem Wasser in Stufe 4 zugegeben wird.

8. Verfahren nach Anspruch 1, bei dem das Produkt aus dem Fermentationsmedium durch Entfernen des Wassers aus dem direkten Fermentationsmedium hergestellt wird.

9. Verfahren nach Anspruch 8, bei dem die genannte Entfernung des Wassers mechanisch erfolgt.

## Revendications

1. Procédé de récupération de natamycine à partir d'un produit de bouillon de fermentation contenant au moins 2g/l de natamycine, comprenant les étapes suivantes :
a. addition de méthanol audit produit de bouillon ;
b. ajustement du pH entre 1,0 et 4,5 pour solubiliser la natamycine ;
c. élimination des solides en suspension ; et
d. augmentation du pH entre 6,0 et 9,0 pour précipiter la natamycine.

2. Procédé de la revendication 1, dans lequel la natamycine précipitée est récupérée et séchée.

3. Procédé de la revendication 1, dans lequel le produit de bouillon de fermentation contient de 20 à 98 % de solides.

4. Procédé de la revendication 1, dans lequel le produit de fermentation contient au moins 50 % de solides.

5. Procédé de la revendication 1, dans lequel le méthanol est ajouté en une quantité de 1 litre de méthanol par 20 g à 150 g de natamycine dans le produit de bouillon de fermentation.

6. Procédé de la revendication 1, dans lequel le méthanol est ajouté en une quantité de 1 litre de méthanol par 20 g à 120 g de natamycine dans le produit de bouillon de fermentation.

7. Procédé de la revendication 1, dans lequel de l'eau est ajoutée dans l'étape 4.

8. Procédé de la revendication 1, dans lequel le produit de bouillon de fermentation est préparé par élimination d'eau du bouillon de fermentation direct.

9. Procédé de la revendication 8, dans lequel ladite élimination d'eau est effectuée par des moyens mécaniques.
